# EUROPEAN PATENT SPECIFICATION

(11) **EP 0 332 405 B1**
(45) Date of publication and mention of the grant of the patent: **25.08.1993**
(21) Application number: 89302276.4
(22) Date of filing: 07.03.1989
(51) Int. Cl.: A61L 25/00, C09J 175/04, C08G 18/77

(54) **Surgical adhesive**
Chirurgischer Klebstoff
Adhésif chirurgical

(30) Priority: 07.03.1988 JP 52918/88
(43) Date of publication of application: 13.09.1989
(73) Proprietor: SANYO CHEMICAL INDUSTRIES LTD., Kyoto 605 (JP); ASAHI GLASS COMPANY LTD., Chiyoda-ku, Tokyo (JP)
(72) Inventor: Matsuda, Takehisa, Minoo-shi Osaka-fu (JP); Takakura, Teruo, Kanagawa-ku Yokohama (JP); Itoh, Tetsuo, Kouka-gun Shiga-ken (JP)
(74) Representative: Marlow, Nicholas Simon

(56) References cited:
- EP-A- 0 073 978
- GB-A- 1 325 429
- US-A- 3 972 856
- US-A- 4 057 535
- US-A- 4 740 534
- J. BIOMED. MATER. RES., vol. 8, no. 1, January 1974, pages 35-43, John Wiley & Sons, Inc.; E. LLEWELLYN-THOMAS et al.: "Adhesion of synthetic organic polymer on soft tissue. I. A fast setting polyurethane adhesive"
- CHEMICAL ABSTRACTS, vol. 69, no. 6, 9th August 1968, page 1863, abstract no. 19645k, Columbus, Ohio, US; R. GOSNELL et al.: "Synthesis of fluorinated polyurethanes"

## Description

This invention relates to surgical adhesive.

Matsuda et al., JP-A-62290465 discloses surgical adhesives, comprising NCO-terminated hydrophilic urethane prepolymers NCO content: 2,5% derived from aromatic polyisocyanates (p-phenylenediisocyanate) and hydrophilic polyether polyols of higher oxyethylene content (80%), or combination thereof with unsaturated cyano compounds containing cyano group attached to a carbon atom constituting the polymerizable double bond.

Such adhesives, however, wherein aromatic polyisocyanates, such as TDI and MDI are used as the raw materials, have some problem, in safety, for tolylene diamine and diaminodiphenylmethane, hydrolyzates of high activity in microbial mutagenicity test (ames test). On the other hand, aliphatic or cycloaliphatic polyisocyanates, such as hexamethylene diisocyanate, are of low reactivity and catalysts such as heavy metal compounds and amines are required for use as surgical adhesives, and use of such catalysts causes problem in safety.

### SUMMARY OF THE INVENTION

It is an object of the present invention to provide a surgical adhesive of lower toxicity.

It is another object of this invention to provide an adhesive for surgery having improved curability.

It is still another object of the invention to provide sufficient bonding power for tissues.

Briefly, these and other objects of the present invention as hereinafter will become more readily apparent have been attained broadly by a surgical adhesive composition comprising [A] at least one NCO-terminated hydrophilic urethane prepolymer having an NCO-content of 1-10%, derived from (a) an organic polyisocyanate component comprising at least one fluorine-containing polyioscyanate or combination thereof with at least one other polyisocyanate and (b) a polyol component having an oxyethylene content of at least 30% by weight and not more than 90% by weight, and comprising at least one hydrophilic polyether polyol having a molecular weight per hydroxyl group fo 100-5,000, in which NCO/OH ratio being 1.5-5.0, and the content of said prepolymer is at least 20% based on the weight of the composition, or a combination of [A] with [B] unsaturated cyano compound containing cyano group attached to a carbon atom constituting the polyermerizable double bond.

### DETAILED DESCRIPTION OF THE PREFERRED EMBODIMENTS

Said NCO-terminated hydrophilic urethane prepolymer [A], used in the surgical adhesive according to the invention, can be derived from (a) at least one organic polyisocyanate comprising a fluorine-containing polyisocyanate and (b) at least one hydrophilic polyether polyol with or without (c) one or more other polyols.

### [F-Containing Polyisocyanates]

Suitable F(fluorine)-containing polyisocyanates, used in producing NCO-terminated hydrophilic urethane prepolymer [A] according to the invention, include, F-containing aliphatic polyisocyanates, and F-containing cycloaliphatic polyisocyanates, and mixtures of two or more of them.

Suitable F-containing aliphatic polyisocyanates include, for example, F-containing diisocyanates, represented by the fomulae (1) and (2):

OCN-Rf-NCO (1)

and

OCN-CH₂-Rf-CH₂-NCO (2)

In the formulae (1) and (2), Rf is a perfluoroalkylene group containing usually 1 to 20 , preferably 1 to 10 carbon atoms, and 0 to 10, preferably 0 - 4 ether linkages. Specifically, there may be mentioned 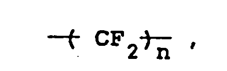
-CF₂CF₂OCF₂CF₂- , -CF₂CF₂O(CF₂)ₘOCF₂CF₂- , -CF(CF₃)O(CF₂)ₘOCF(CF₃)- , and -CF₂CF₂OCF₂CF(CF₃)O(CF₂)ₘOCF(CF₃)CF₂OCF₂CF₂-, wherein n is 1-20, preferably 1-10, m is 1-5, preferably 1-2. Among these, preferred are 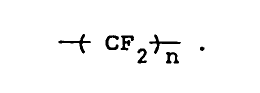
F-containing polyisocyanates can be produced according to the methods described in J.Macromol.Sci.-Phys.,B1, 831('67) and JPN Lay-open Patent No. 108055 /1982.

Illustrative examples of F-containing cycloaliphatic polyisocyanates are fluorinated cycloaliphatic polyisocyanates, containing 4 - 15 carbon atoms (except those in NCO groups), such as F-containing isophorone diisocyanates, fluorinated hydrogenated xylylene diisocyanates, fluorinated hydrogenated 4,4′-diphenylmethane diisocyanates and fluorinated trans-cyclohexane-1,4-diisocyanates.

Among these polyisocyanates, preferred are F-containing aliphatic polyisocyanates, particularly F-containing diisocyanates of the fomula (2). The most preferred is 2,2,3,3,4,4,5,5- octafluorohexamethylene diisocyanate (hereinafter referred to as FHDI).

### [Other Polyisocyanates]

F-containing polyisocyanates may be used alone or in combination with other polyisocyanates. Suitable examples of such polyisocyanates include aromatic polyisocyanates containing 6 - 20 carbon atoms [except carbon atoms in NCO groups], such as o-, m- and p-phenylene diisocyanates [hereinafter referred to as PDI], 2,4- and 2,6-tolylene diisocyanates [TDI], diphenylmethane-2,4′-and 4,4′-diisocyanates [MDI], naphthalene-1,5-diisocyanate, triphenylmethane-4,4′,4˝-triisocyanate, polyethylene polyphenylenepolyisocyanates [PAPI] obtained by phosgenation of aniline-formldehyde condensation products, m- and p-isocyanato-phenyl sulfonyl isocyanate, and the like; aliphatic polyisocyanates containing 2 - 18 carbon atoms, such as ethylenediisocyanate, tetramethylenediisocyanate, hexamethylenediisocyanate (hereinafter referred to as HDI), dodecamethylenediisocyanate, 1,6,11-undecane diisocyanate, 2,2,4-trimethylhexanediisocyanate, lysine diisocyanate, 2,6-diisocyanato-methyl caproate, bis(2-isocyanatoethyl fumarate, bis(2-isocyanatoethyl) carbonate, 2-isocyanatoethyl-2,6-diisocyanato hexanoate, and the like; alicyclic polyisocyanates containing 4 - 15 carbon atoms, such as isophorone diisocyanate, dicyclohexylmethane diisocyanates, cyclohexylene diisocyanates, methylcyclohexylene diisocyanates, bis(2-isocyanato-ethyl) 4-cyclohexene-1,2-dicarboxylate, and the like; araliphatic polyisocyanates containing 8 - 15 carbon atoms, such as xylylene diisocyanates, diethylbenzene diisocyanates, and the like; and modified polyisocyanates of these polyisocyanates, containing urethane, carbodiimide, allophanate, urea, biuret, urethdione, urethimine, isocyanurate and/or oxazolidone groups, such as urethane-modified TDI, carbodiimide-modified MDI, urethane-modified MDI, and the like; as well as mixtures of two or more of them. Among these polyisocyanates, preferred are aromatic polyisocyanates (preferably diisocyanates), particularly PDI, TDI (including 2,4- and 2,6-isomers, mixtures of them and crude TDI), MDI (including 4,4′-and 2,4′-isomers, mixtures of them and crude MDI or PAPI), and modified polyisocyanates ontaining urethane, carbodiimide, allophanate, urea, biuret and/or isocyanurate groups, derived from PDI, TDI and/or MDI. The most preferred is p-PDI, with respect to low toxicity.

These other polyisocyanates may be used in an amount of usually 80 % or less, preferably 50 % or less, more preferably 20 % or less, based on the total weight of the polyisocyanates.

### [Hydrophilic Polyether Polyols]

Illustrative of suitable hydrophilic polyether polyols (b) are adducts of ethylene oxide [ hereinafter referred to as EO ] or combinations thereof with other alkylene oxide(s) [hereinafter refferred to as AO] to one or more compounds containing at least two active hydrogen atoms, such as polyhydric alcohols, polyhydric phenols, amines, polycarboxylic acids, phosphorous acids and the like. Suitable examples of polyhydric alcohols include dihydric alcohols, such as ethylene glycol, propylene glycol, 1,3-and 1,4-butane diols, 1,6-hexane diol, neopentyl glycol, diethylene glycol, bis(hydroxymethyl)cyclohexane, bis(hydroxyethyl)benzene, hydrogenated bisphenol A, hydrogenated bisphenol F, polytetramethylene glycols, polyester diols and silanol-terminated polysiloxanes; trihydric alcohols, such as glycerol, trimethylol propane, trimethylol ethane, 1,2,3-butane triol, 1,2,6-hexane triol and polyester triols; and polyhydric alcohols having 4 - 8 or more hydroxyl groups, such as pentaerythritol, diglycerol, alpha-methylglucoside, sorbitol, xylitol, mannitol, glucose, fructose, sucrose, and the like. Exemplary of suitable polyhydric phenols are mono- and poly-nuclear phenols, such as hydroquinone, catechol, resorcin, pyrogallol, and bisphenols (bisphenol A, bisphenol F, bisphenol S and the like), as well as phenol-formaldehyde condensation products. Suitable amines are inclusive of ammonia; alkanol amines, such as mono-, di- and tri- ethanol amines, isopropanol amines and the like; aliphatic, aromatic, araliphatic and alicyclic monoamines, such as C₁-C₂₀ alkyl amines [methyl, ethyl, isopropyl, butyl, octyl and lauryl amines, and the like], aniline, toluidine, naphthyl amines, benzyl amine, cyclohexyl amine and the like, aliphatic, aromatic, alicyclic and araliphatic polyamines, such as C₂-C₆ alkylene diamines [ethylene diamines], diethylene triamine, tolylene diamines, phenylene diamines, xylylene diamines, methylene dianilines, diphenylether diamines, isophorone diamine, cyclohexylene diamines, dicyclohexylmethane diamines and the like; and heterocyclic polyamines, such as piperazine, N-aminoethyl-piperazine, and other heterocyclic polyamines, written in Japan Patent Publication No. 21044 / 1980.

Suitable AO, which may be employed in combination with EO for producing polyether polyols, include, for example, propylene oxide [hereinafter referred to as PO] , 1,2- 2,3- ,1,3- and 1,4-butylene oxides, styrene oxide, epichlorohydrin and the like, as well as combinations of two or more of them. Among these, preferred are PO.

Addition of EO or combination thereof with AO to active hydrogen atom-containing compounds can be carried out in the usual way, with or without catalysts [such as alkaline catalysts, amine catalysts and acidic catalysts], under normal or an elevated pressure, in a single step or multi-stages. Addition of EO and AO may be performed by random-addition, block-addition or combination of them (for instance random-addition followed by block-addition). Preferred is random-addition.

Hydrophilic polyether polyols have equivalent weight (molecular weight per hydroxyl group) of usually 100 - 5,000, preferably 200 - 3,000, and oxyethylene content of usually at least 30%, preferably 50 - 90 % by weight. Polyether polyols having equivalent weight higher than 5,000 are too viscous to be used as surgical adhesives; while equivalent weight less than 100 results in lack of flexibility required for surgical adhesives. Polyether polyols of oxyethylene content less than 30 % by weight, having insufficient hydrophilic nature, have poor reactivity with body fluids resulting in reduced cure rate and poor bonding power with water-rich tissue. Content of the primary hydroxyl groups of polyether polyols is preferably at least 30 %, more preferably at least 50 %, most preferably at least 70 %.

### [Other Polyols]

Other polyols (c), optionally used in conjunction with hydrophilic polyether polyols, include low molecular weight polyols and/or hydrophobic polyols. Examples of such polyols are polyhydric alcohols mentioned above (as raw materials for hydrophilic polyether polyols); AO adducts (such as PO adducts) of these polyhydric alcohols or other active hydrogen atom-containing compounds; and polyester polyols. Illustrative examples of polyester polyols are condensation products of dihydric and/or trihydric alcohols (ethylene glycol, propylene glycol, 1,3- and 1,4-butane diols, 1,6-hexane diol, neopentyl glycol, diethylene glycol, glycerol, trimethylolpropane and the like) and/or polyether polyols (such as those described above) with dicarboxylic acids (aliphatic or aromatic dicarboxylic acids, such as glutaric, adipic, sebacic, fumaric, maleic, phthalic and terephthalic acids) or ester-forming derivatives thereof (anhydrides and lower alkyl esters, such as maleic and phthalic anhydrides, dimethyl terephtharate, and the like); ring-opening polymerization products of lactones [such as epsilon-caprolactone]. Among these polyols, polyether polyols are preferred to polyester polyols.

The whole polyols [(b) and optionally (c)], used for producing NCO-terminated urethane prepolymer, have oxyethylene content of usually at least 30 %, preferably 50 - 90 % by weight, equivalent weight (average) of usually 100 - 5,000, preferably 200 - 3,000, and usually 2 - 8 hydroxyl groups, preferably 2 - 4 hydroxyl groups.

### [Prepolymer]

In reacting at least one polyisocyanate (a) comprising F-containing polyisocyanate with at least one hydrophilic polyether polyol (b) and optionally one or more other polyols (c) to form NCO-terminated hydrophlic urethane prepolymers, ratio of NCO/OH is generally 1.5 - 5.0, preferably 1.7 - 3.0. The reaction of (a) with (b) and optionally (c) forming prepolymers can be performed in the usual manner. The reaction may be carried out in the presence of a catalyst. Prepolymers may be prepared by reacting (a) with a mixture of (b) and (c), or reacting successively in any order with (b) and (c). Prepolymers may be prepared by blending a prepolymer from (b) with a prepolymer from (c) [for instance, blending with a prepolymer from a low molecular weight polyol (equivalent weight 50 - 500) to reduce viscosity]. In case where F-containing polyisocyanate is used in conjunction with one or more other polyisocyanates, these polyisocyanates can be reacted in any order, but it is preferred to react said other polyisocyanates at early stages of prepolymer production so as to provide F-containing polyisocyanate-terminated prepolymers.

NCO-contents of NCO-terminated hydrophilic prepolymers are usually 1 - 10%, preferably 2 - 8 % by weight. Prepolymers of NCO-content less than 1 % by weight are of poor reactivity and bring about reduction of cure rate and insufficient bonding power to tissues. Higher NCO-content than 10 % by weight results in brittle cured resins of poor flexibility which are not deformable following the movement of living organism.

### [Unsaturated Cyano Compounds]

Illustrative examples of suitable unsaturated cyano compound [B] containing cyano group attached to a carbon atom constituting the polymerizable double bond are cyano(meth)acrylic acids [ cyanoacrylic acid and cyanomethacrylic acid; similar expressions are used hereinafter ]; cyano(meth)acrylic esters, such as methyl cyano(meth)acrylates, ethyl cyano(meth)acrylates, iso-butyl cyano(meth)acrylates and the like; (meth)acrylonitriles, cyano(meth)acrylonitriles, and the like; and mixtures of two or more of these compounds. Among these, preferred are cyano-acrylic esters, especially methyl cyanoacrylate, ethyl cyanoacrylate and iso-butyl cyanoacrylate.

In adhesive compositions comprising said hydrophilic urethane prepolymer [A] and said unsaturated cyano compound [B] , content of [A] is usually at least 20 %, preferably 50 - 95 %, more preferably 70 - 90 %, based on the total weight of [A] and [B]. Use of less than 20 % of [A] results in poor flexibility and poor bonding ability with living organism. Combination of [A] with 10% or more of [B] provide more rapid cure rate, which can be applied for bonding of blood vessels, wherein quick-curing is required. By varying the ratio of [A] to [B], there can be attained desired hardness in accordance with movement of living organism. Adhesives containing higher amount of [A] are effective for applications requiring flexibility, such as bonding of blood vessels; while adhesives of lower content of [A], providing relatively higher rigidity, are suitable for bonding of bone or circumference thereof.

Adhesives of this invention may contain, if necessary, physiologically actitive mateials [such as antimicrobias, ocal anesthetics, antihistamines, antiphlogosis analgestics, antibiotics, astringents, vitamins, antifungal agents, Peripheral nervous anesthetics, vasodilators, hormones, crude drug essences, tinctures, crude drug powders, hypotensive agents, and the like], fillers [for example, carbon black, metal oxides, such as red iron oxide and titanium dioxide, silicates, such as calcium silicates and sodium silicates, acrylic resin powders, various ceramic powders, and the like]; softening agents [such as DBP(dibutylphosphate), DOP(dioctylphosphate), TCP(tricresylphosphate), tributoxyethylphosphates, and other esters of various types]; stabilizers, such as trimethyldihydroquinone, phenyl-beta-naphthyl amine, p-isopropoxydiphenylamine, diphenyl-p-phenylene diamine, and the like. These additives may be used in an amounts of usually 0 - 20 %, preferably 0 - 5%, based on the weight of the adhesive according to the invention.

Both said NCO-terminated prepolymer(s) and said cyano compound(s) can bring about rapid polymerization in the presence of trace amounts of water such as moisuture in air and result in forming tough membrane. Accordingly, it is necessary to use dehydrated ones as these main components and also other compounding additives, and it is preferred to shut off air during production of adhesives. Adhesives, thus obtained, can be stored for a long period of time within airtight vessels, such as ampule.

### [Application of Surgical Adhesive]

In applying adhesives of the present invention in surgery, application methods include those by using brushes, tweezers, applicators, specially-designed spatula or syringes, or the like; and those by spray coating using innert gases, such as Freons, nitrogen or the like. Bonding of tissues can be achieved, for example, by direct coating techniques, simply applying the adhesive to the tissues; by cover-coating techniques, using, as an aid for hemostasis or anastomosis, thin sheets or meshes made of polyesters ( such as Dacron ), oxidized celllose, collagen, polyurethanes or the like, cotton-like materials, or fragments of tissues, such as veins, musculation or mascular membrane or the like [ wherein these materials are applied onto the affected parts followed by coating thereon the adhesives ]; or by sealing techniques for sutured parts, wherein sutures are partly applied followed by applying the adhesive to seal the remaining conjugation parts. The adhesives of the invention can be used, not only for tissure adhesion, but also as coating, embolus or sealing materials in cardiovascular surgery via direct coating or injection by catheters. Applicable tissues include, for example, vascular vessels, heart, lung, esophagus, stomach, liver, pancreas, spleen, skin and the like.

Surgical adhesives according to this invention, comprising NCO-terminated hydrophilic urethane prepolymer [A] derived from F-containing polyisocyanate and hydrophilic polyether polyol, have sufficiently high cure rate and provide sufficient bonding power for tissues, even without any catalyst, in spite of using F-containing polyisocyanates of aliphatic polyisocyanate type as the raw material; while usual aliphatic polyisocyanates are of low reactivity and in need of catalysts such as heavy metal compounds and amines, which cause problem in safety. Surgical adhesives of the invention cause no or little toxicologicai problem, since F-containing polyisocyanates used therein show no multagenic activity; whereas aromatic polyisocyanates, such as TDI and MDI, show generally high multagenic activity and have possibility of carcinogenesis during use within tissues for long period of time. In addition, surgical adhesives of this invention are elastomeric of flexible and can be deformed in accordance with movement of tissues.

Surgical adhesives of the invention, comprising said prepolymer [A] and unsaturated cyano compound [B], can attain cure accelerating effects in entirety (not only tissue contact surface but also inside of adhesive), shortening of operation period and increase in over all bonding power with body tissue, by synergistic action of rapid polymerization of [B] in contact with body fluid (water) along with reactivity of NCO groups of [A].

In addition, adhesives of the invention can provide sufficient cure rate, bonding power to tissue and flexibility follow-up to body movement, required for surgical adhesives, without using any organic solvents, which may cause problem in safety.

Accordingly, application of adhesives of the present invention to surgical operation makes it possible to perform operation with method of adhesion, instead of suturing in conventional operation. There can be attained remarkable improvements in medical technology, such as shortening of operation time, hemostasis, prevention of leaking emzyme from viscera or the like, prevention of minute bood bessel occusion, and nerve anastomosis, as well as provisional fixing before suturing, and ensuring of bonding by combination of adhesion with suturing. Furthermore, the invention can provide high reliance and high efficiency, not only in operation, but also in medical treatment at large, for example, joining of incised wound or culting portions, adhesive treatment in dental surgery, curative means by controled release of drugs in combination with physiologically active materials, and so on.

Having generally described the invention, a more complete understanding can be obtained by reference to certain specific examples, which are included for purposes of illustration only and are not intended to be limiting unless otherwise specified.

In the following, parts and % represent parts by weight and % by weight, respectively. Raw materials used in the following examples are as follows :
PEO : Polyethyleneoxide, PPO : Polypropyleneoxide,
PEG : polyetheleneglycol, PPG : Polypropyleneglycol,
PTMG : Polytetramethyleneglycol,
ECA : Ethyl cyanoacrylate.

### [Preparation of Prepolymers]

NCO-terminated prepolymers A1 to A4, and B1 to B4 were prepared by mixing each polyether polyol, dehydrated under reduced pressure, with each polyisocyanate, and reacting them for 8 hours at 80°C.

### Prepolymer A1

A polyether polyol [a EO/PO random copolymer having an average M.W. of 3,000 and oxyethylene content of 80%] was reacted with FHDI to obtain a NCO-terminated hydrophilic urethane prepolymer having an NCO-content of 2.5%.

### Prepolymer A2

A polyether polyol [a EO/PO random copolymer having an average M.W. of 4,000 and oxyethylene content of 60%] was reacted with FHDI to obtain a NCO-terminated hydrophilic urethane prepolymer having an NCO-content of 3.4%.

### Prepolymer A3

A polyether polyol [a mixture of 80 parts of a PEG having an average M.W. of 2,000 and 20 parts of PPG having an average M.W. of 200] was reacted with FHDI to obtain a NCO-terminated hydrophilic urethane prepolymer having an NCO-content of 6.4%.

### Prepolymer A4

A polyether polyol [a PTMG-EO block copolymer having an average M.W. of 2,000 and oxyethylene content of 50%] was reacted with FHDI to obtain a NCO-terminated hydrophilic urethane prepolymer having an NCO-content of 6.7%.

### Prepolymer B1

A polyether polyol [a EO/PO random copolymer having an average M.W. of 3,000 and oxyethylene content of 80%] was reacted with TDI to obtain a NCO-terminated hydrophilic urethane prepolymer having an NCO-content of 2.5%.

### Prepolymer B2

A polyether polyol [a EO/PO random copolymer having an average M.W. of 3,000 and oxyethylene content of 10%] was reacted with FHDI to obtain a NCO-terminated hydrophobic urethane prepolymer having an NCO-content of 2.5%.

### Prepolymer B3

A polyether polyol [a EO/PO random copolymer having an average M.W. of 4,000 and oxyethylene content of 60%] was reacted with HDI to obtain a NCO-terminated hydrophilic urethane prepolymer having an NCO-content of 3.4%.

### Prepolymer B4

A polyether polyol [a mixture of 80 parts of a PEG having an average M.W. of 2,000 and 20 parts of PPG having an average M.W. of 200] was reacted with MDI to obtain a NCO-terminated hydrophilic urethane prepolymer having an NCO-content of 6.4%.

### [ Preparation of Surgical Adhesives ]

Surgical adhesives as follows were prepared.

### Examples 1 to 4

Surgical adhesives consisting essentially of Prepolymers A1, A2, A3 and A4, respectively.

### Example 5

A surgical adhesive, obtained by mixing and dehydrating 70 parts of Prepolymer A1 with 30 parts of ECA.

### Comparative Examples 1 to 4

Surgical adhesives, obtained by substituting Prepolymers B1, B2, B3 and B4 for Prepolymers A1, A2, A3 and A4, respectively.

### Comparative Example 5

A surgical adhesive consisting essentially of ECA.

### Comparative Example 6

A surgical adhesive, obtained by dissolving 7 parts of a nitrile rubber (nitrile content : 38-40%) into 50 parts of dehydrated dry nitromethane, followed by adding thereto under stirring 7 parts of ECA and 1 part of TDI.

### [ Testing of Surgical Adhesives ]

Carotid artery of goat was cramped at about 5 cm of distance and then incised 3 mm along with the longitudinal direction, followed by coating a small amount of each adhesive. Within several minutes after application of the adhesive, the cramps were removed. Then, the tissue adhesivity and hemostasis were carefully evaluated. Testing was carried out under hepalinized anti-coagulation conditions, in order to exclude stanching effects of blood coagulation to evaluate effects of adhesives. The results were as shouwn in Table 1.

### Method for Safety Test

Safety test was evaluated by microbial mutagenicity test (ames test). Hydrolyzate (diamine) of surgical adhesive was used as the testing sample. Salmonella typhimurium and Escherichia coli were used as testing bacteriums. Test results were shown in Table 1 according to the following criterion :
- - :: negative (no mutagenic activity was observed);
- + :: positive (mutagenic activity was observed).

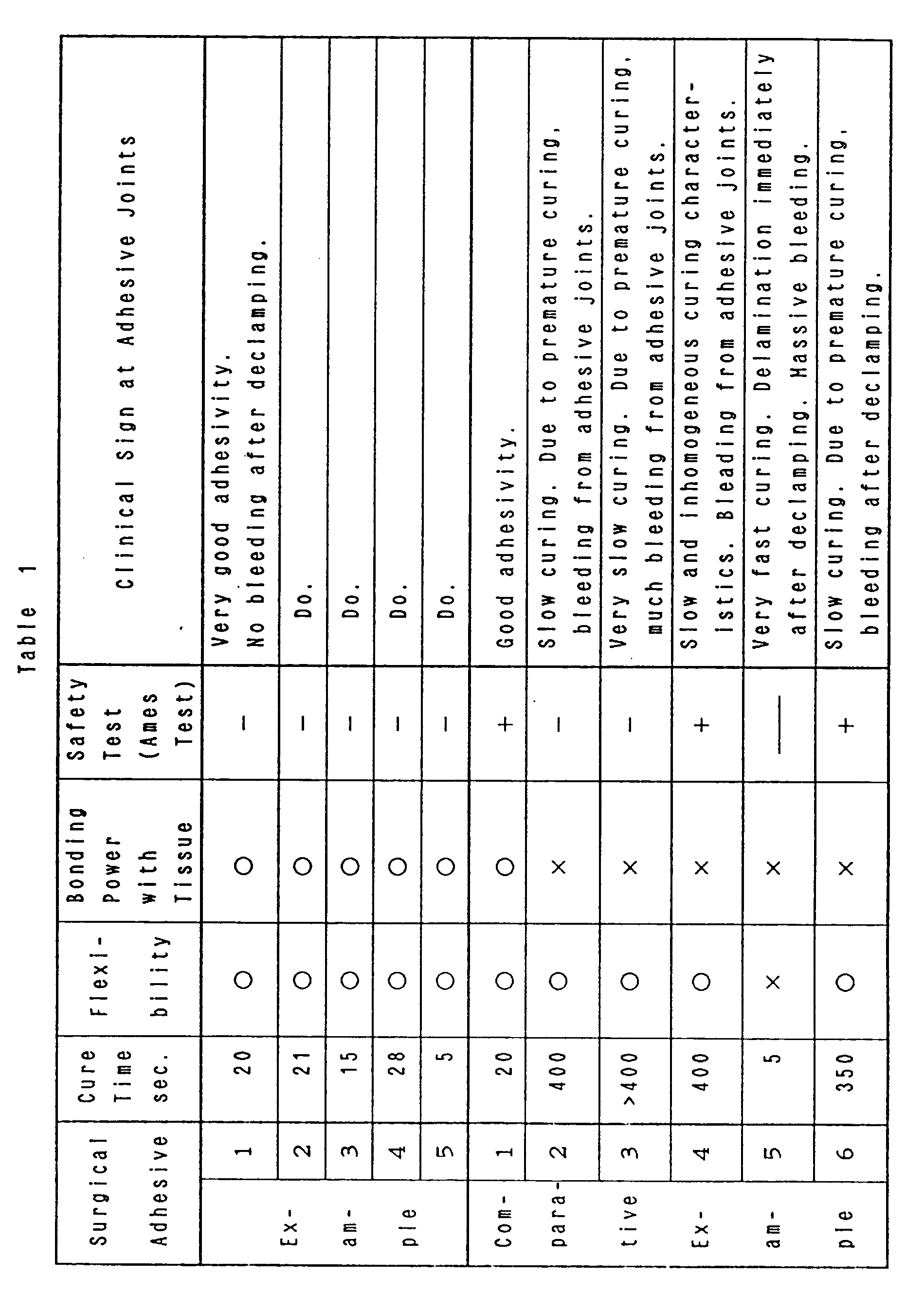

## Claims

1. A surgical adhesive composition comprising at least one NCO-terminated hydrophilic urethane prepolymer having an NCO-content of 1-10%, derived from (a) an organic polyisocyanate component comprising at least one fluorine-containing polyioscyanate or combination thereof with at least one other polyisocyanate and (b) a polyol component having an oxyethylene content of at least 30% by weight and not more than 90% by weight, and comprising at least one hydrophilic polyether polyol having a molecular weight per hydroxyl group of 100 to 5,000, in which the NCO/OH ratio is 1.5 to 5.0, and the prepolymer is present in an amount of at least 20% based on the weight of the composition.

2. A composition according to claim 1, in which the fluorine-containing polyisocyanate is at least one fluorine-containing aliphatic polyisocyanate or fluorine-containing cycloaliphatic polysiocyanate.

3. A composition according to claim 1, in which the fluorine-containing polyisocyanate is at least one fluorine-containing diisocyanate of the formula (1) or (2):
OCN-Rf-NCO (1)
OCN-CH₂-Rf-CH₂-NCO (2)
in which Rf is a perfluoroalkylene group containing usually from 1 to 20 carbon atoms and from 0 to 10 ether linkages.

4. A composition according to claim 1, 2 or 3 in which the prepolymer has an isocyanate-content of from 2 to 8% by weight.

5. A composition according to any of claims 1 to 4 in which the hydrophilic polyether polyol is at least one adduct of ethylene oxide or a combination thereof with one or more other alkylene oxides with at least one compound containing two or more active hydrogen atoms.

6. A composition according to any of claims 1 to 5 in which the polyol component comprises the hydrophilic polyether polyol and at least one low molecular weight polyol, hydrophobic polyether polyol or polyester polyol.

7. A composition according to any of claims 1 to 6 in which the polyol component has an oxyethylene content of between 50% and 90%.

8. A composition according to any of claims 1 to 7 in which the polyol component has an equivalent weight (average) of 100 to 5,000 and a functionality (average) of from 2 to 8.

9. A composition according to any one of claims 1 to 8 in which the prepolymer is obtained by reacting the polyisocyanate with polyol component in such an amount as to provide an NCO/OH ratio of between 1.7 and 3.0.

10. A composition according to any of claims 1 to 9 comprising
[A] the prepolymer, and
[B] at least one unsaturated cyano compound containing a cyano group attached to a carbon atom constituting the polymerizable double bond.

11. A composition according to claim 10 comprising between 20 and 90% by weight of the prepolymer and between 10 and 80% by weight of the cyano compound.

12. A composition according to any of claims 1 to 11 containing up to 20% by weight of at least one of carbon black, metal oxides, silicates, acrylic resin powders, ceramic powders, softening agents, stabilizers and physiologically active materials.

13. Use of a composition according to any of claims 1 to 12 as surgical adhesive.

## Patentansprüche

1. Chirurgische Klebstoff-Zusammensetzung, umfassend mindestens ein NCO-terminiertes, hydrophiles Urethan-Prepolymer mit einem NCO-Gehalt von 1-10%, das (a) von einer organischen Polyisocyanat-Komponente, die mindestens ein Fluor enthaltendes Polyisocyanat oder eine Kombination davon mit mindestens einem weiteren Polyisocyanat umfaßt, und (b) von einer Polyol-Komponente mit einem Oxyethylen-Gehalt von mindestens 30 Gew.-% und nicht mehr als 90 Gew.-% stammt, und umfassend mindestens ein hydrophiles Polyetherpolyol mit einem Molekulargewicht pro Hydroxyl-Rest von 100 bis 5000, in dem das NCO/OH-Verhältnis 1,5 bis 5,0 beträgt, wobei das Prepolymer in einer Menge von mindestens 20 %, basierend auf dem Gewicht der Zusammensetzung vorliegt.

2. Zusammensetzung nach Anspruch 1, worin das Fluor enthaltende Polyisocyanat mindestens ein Fluor enthaltendes, aliphatisches Polyisocyanat oder ein Fluor enthaltendes, cycloaliphatisches Polyisocyanat ist.

3. Zusammensetzung nach Anspruch 1, worin das Fluor enthaltende Polyisocyanat mindestens ein Fluor enthaltendes Diisocyanat der Formel (1) oder (2):
OCN-Rf-NCO (1)
OCN-CH₂-Rf-CH₂-NCO (2)
ist, worin Rf ein Perfluoralkylen-Rest mit üblicherweise 1 bis 20 C-Atomen und 0 bis 10 Etherbrücken ist.

4. Zusammensetzung nach Anspruch 1, 2 oder 3, worin das Prepolymer einen Isocyanat-Gehalt von 2 bis 8 Gew.-% aufweist.

5. Zusammensetzung nach einem der Ansprüche 1 bis 4, worin das hydrophile Polyetherpolyol mindestens ein Additionsproduct von Ethylenoxid oder einer Kombination aus ihm und ein oder mehreren anderen Alkylenoxiden mit mindestens einer, zwei oder mehr aktive Wasserstoffatome enthaltenden Verbindung.

6. Zusammensetzung nach einem der Ansprüche 1 bis 5, worin die Polyol-Komponente das hydrophile Polyetherpolyol und mindestens ein Polyol niedrigen Molekulargewichts, ein hydrophobes Polyetherpolyol oder Polyesterpolyol umfaßt.

7. Zusammensetzung nach einem der Ansprüche 1 bis 6, worin die Polyol-Komponente einen Oxyethylen-Gehalt von zwischen 50 % und 90 % aufweist.

8. Zusammensetzung nach einem der Ansprüche 1 bis 7, worin die Polyol-Komponente ein (Durchschnitts)-Equivalentgewicht von 100 bis 5000 und eine (Durchschnitts)-Funktionalität von 2 bis 8 hat.

9. Zusammensetzung nach einem der Ansprüche 1 bis 8, worin das Prepolymer erhalten wird, indem das Polyisocyanat mit der Polyol-Komponente in einer solchen Menge umgesetzt wird, daß ein NCO/OH-Verhältnis von zwischen 1,7 und 3,0 erhalten wird.

10. Zusammensetzung nach einem der Ansprüche 1 bis 9, umfassend
[A] das Prepolymer, und
[B] mindestens eine ungesättigte Cyano-Verbindung mit einem Cyano-Rest, der an ein die polymerisierbare Doppelbindung ausbildendes C-Atom gebunden ist.

11. Zusammensetzung nach Anspruch 10, umfassend 20 bis 90 Gew.-% des Prepolymers und 10 bis 80 Gew.-% der Cyano-Verbindung.

12. Zusammensetzung nach einem der Ansprüche 1 bis 11 mit bis zu 20 Gew.-% von mindestens einem von Kohle schwarz, Metalloxiden, Silikaten, Acrylsäureharzpulvern, keramischen Pulvern, Weichmachern, Stabilisatoren und physiologisch aktiven Materialien.

13. Verwendung einer Zusammensetzung nach einer der Ansprüche 1 bis 12 als chirurgischer Klebstoff.

## Revendications

1. Composition d'adhésif chirurgical comprenant au moins un prépolymère d'uréthanne hydrophile à terminaison NCO ayant une teneur en NCO de 1 à 10 %, dérivé (a) d'un constituant du type polyisocyanate organique comprenant au moins un polyisocyanate contenant du fluor ou une association d'un tel polyisocyanate avec au moins un autre polyisocyanate, et (b) un constituant du type polyol ayant une teneur en groupes oxyéthylène d'au moins 30 % en poids et non supérieure à 90 % en poids, et comprenant au moins un polyéther-polyol hydrophile ayant un poids moléculaire, par groupe hydroxyle, de 100 à 5000, dans laquelle le rapport NCO/OH va de 1,5 à 5,0, et le prépolymère est présent en une quantité d'au moins 20 % sur la base du poids de la composition.

2. Composition suivant la revendication 1, dans laquelle le polyisocyanate contenant du fluor consiste en au moins un polyisocyanate aliphatique contenant du fluor ou un polyisocyanate cycloaliphatique contenant du fluor.

3. Composition suivant la revendication 1, dans laquelle le polyisocyanate contenant du fluor consiste en au moins un diisocyanate contenant du fluor répondant à la formule (1) ou (2) :
OCN-Rf-NCO (1)
OCN-CH₂-Rf-CH₂-NCO (2)
dans laquelle Rf représente un groupe perfluoralkylène contenant habituellement 1 à 20 atomes de carbone et 0 à 10 liaisons éther.

4. Composition suivant la revendication 1, 2 ou 3, dans laquelle le prépolymère possède une teneur en groupes isocyanate de 2 à 8 % en poids.

5. Composition suivant l'une quelconque des revendications 1 à 4, dans laquelle le polyéther-polyol hydrophile consiste en au moins un produit d'addition d'oxyde d'éthylène ou d'une association d'oxyde d'éthylène avec un ou plusieurs autres oxydes d'alkylène avec au moins un composé contenant deux ou plus de deux atomes d'hydrogène actif.

6. Composition suivant l'une quelconque des revendications 1 à 5, dans laquelle le constituant du type polyol comprend le polyéther-polyol hydrophile et au moins un polyol de bas poids moléculaire, un polyéther-polyol hydrophobe ou un polyester-polyol.

7. Composition suivant l'une quelconque des revendications 1 à 6, dans laquelle le constituant du type polyol possède une teneur en groupes oxyéthylène de 50 % à 90 %.

8. Composition suivant l'une quelconque des revendications 1 à 7, dans laquelle le constituant du type polyol possède un poids équivalent (valeur moyenne) de 100 à 5000 et une fonctionnalité (moyenne) de 2 à 8.

9. Composition suivant l'une quelconque des revendications 1 à 8, dans laquelle le prépolymère est obtenu par réaction du polyisocyanate avec le constituant du type polyol en une quantité choisie de manière à parvenir à un rapport NCO/OH compris dans l'intervalle de 1,7 à 3,0.

10. Composition suivant l'une quelconque des revendications 1 à 9, comprenant
[A] le prépolymère, et
[B] au moins un composé insaturé à fonction cyano, contenant un groupe cyano fixé à un atome de carbone constituant la double liaison polymérisable.

11. Composition suivant la revendication 10, comprenant 20 à 90 % en poids du prépolymère et 10 à 80 % en poids du composé à fonction cyano.

12. Composition suivant l'une quelconque des revendications 1 à 11, contenant jusqu'à 20 % en poids d'au moins un composé choisi entre le noir de carbone, des oxydes métalliques, des silicates, des poudres de résines acryliques, des poudres céramiques, des agents de ramollissement, des stabilisants et des substances physiologiquement actives.

13. Utilisation d'une composition suivant l'une quelconque des revendications 1 à 12 comme adhésif chirurgical.
